Europäisches Patentamt

European Patent Office

Office européen des brevets

(11) Publication number: 0 394 021
A2

# (12) EUROPEAN PATENT APPLICATION

(21) Application number: 90304168.9

(22) Date of filing: 18.04.90

(51) Int. Cl.5: G01N 33/545, G01N 33/52,
//G01N33/53

(30) Priority: 19.04.89 US 340244

(43) Date of publication of application:
24.10.90 Bulletin 90/43

(84) Designated Contracting States:
DE FR IT

(71) Applicant: PALL CORPORATION
30 Sea Cliff Avenue
Glen Cove New York 11542(US)

(72) Inventor: Harwood, Colin F.
17 Southland Drive
Glen Cove, New York 11542(US)
Inventor: Matkovich, Vlado I.
11 Old Estate Road
Glen Cove, New York 11542(US)
Inventor: Krasnoff, Abraham
Beech House, Valley Road
Glen Cove, New York 11542(US)

(74) Representative: Knott, Stephen Gilbert et al
MATHISEN, MACARA & CO. The Coach
House 6-8 Swakeleys Road
Ickenham Uxbridge Middlesex UB10 8BZ(GB)

(54) Diagnostic device with porous, absorbent single element.

(57) A diagnostic device is provided which comprises a receptacle having an open bottom; and a liquid absorbent, wettable, porous structure formed from a medium having a low non-specific affinity for amide group-containing substances, as measured by the Immersion Protein Binding Assay, secured to the receptacle and disposed across the open bottom.

EP 0 394 021 A2

## DIAGNOSTIC DEVICE WITH POROUS, ABSORBENT SINGLE ELEMENT

The present invention is directed to a diagnostic device capable of producing uniform and reproducible test results. More particularly, the present invention is directed to a diagnostic device which includes a porous structure which assures rapid and uniform unassisted removal of liquid from a test zone.

In recent years, medical research has led to a proliferation of diagnostic tests and techniques as well as the apparatus and devices employed to carry out such tests. ELISA assays, RNA and DNA hybridization assays, and microbiological assays are but a few of the types of diagnostic test methods which have been developed recently. In addition to providing a diagnostic test procedure for a particular bacteria, virus, antigen, etc., greater sensitivity, accuracy (fewer false positives), rapidity, and simplification have also been objectives sought in developing test procedures and devices. Such devices have frequently taken the form of a reservoir or receptacle in the form of a tube or well, having a porous structure, typically a membrane, secured to an open bottom portion of the reservoir. This porous or microporous membrane generally functions in one of two ways: (1) it acts as a reaction layer in which a test reagent immobilized on or within the microporous membrane provides an indicia of the presence or absence of a specific analyte, or (2) it may function to retain on its upper surface small test particles, typically plastic particles, such as polystyrene spheres or beads which are provided with a test reagent on their surfaces for the detection of an analyte.

To provide maximum sensitivity, whether using a membrane in which the test reagent is immobilized at its surface (type (1)) or reagent-containing plastic beads are retained thereon (type (2)), maximum surface area of the membrane is sought. Thus, with devices of type (1), the higher the surface area the more test reagent may be immobilized on the surface and the higher the sensitivity. This is achieved by employing a microporous membrane. With devices of type (2), surface area varies inversely with particle size of the beads employed. As smaller particles are employed to achieve high surface area, the pores of the membrane used are, accordingly, smaller, or at least the surface adjacent the beads has smaller pores, to avoid penetration of the pores by the beads. Thus, the membrane is also microporous or the gross surface adjacent the beads is microporous.

Although in some instances it is necessary to allow either a suspected analyte-containing liquid or a reagent-containing liquid to remain in the well above the surface of the membrane to assure adequate incubation or development of a reaction product which indicates a positive test, it is also necessary in many instances to remove the liquid from the reservoir at a controlled and, preferably, rapid rate. Liquid removal may be achieved in a number of ways. Each method, however, requires some driving or motive force which induces or forces liquid through the membrane. Typically these methods involve principles of gravity flow, differential pressure, or wicking. The first method, employing gravity flow, may be effective if the height of the reservoir or tube is sufficiently great to accommodate a tall column of fluid and/or the pores of the membrane are sufficiently large. However, with the above-discussed requirements of surface area and the use of a microporous membrane, along with the practical considerations of using a tube or well with a relatively short height in order to facilitate transfer of liquids and permit ease of viewing the membrane or beads positioned on the upper surface of the membrane, the height of the reservoir is typically too short to permit unassisted gravity flow of liquid through a microporous membrane at a reasonable rate.

The use of either elevated pressure or, more commonly, vacuum equipment to effect a differential pressure across the membrane in a diagnostic device in which such membrane is located at the bottom of a liquid reservoir provides an effective method of removing liquid from the reservoir. However, this technique requires somewhat elaborate equipment such as a pump to create an elevated pressure or a "source" of vacuum and a manifold, such as a vacuum manifold, to hold the reservoir containing devices, such as tubes or a well-containing plate, and to maintain the desired pressure. Although many laboratories may have such equipment, there are some which do not and the requirement of such equipment forecloses the ability to perform simple diagnostic tests in the home or small laboratory. In addition, although not demanding a high skill level, the use of such equipment does require the user to have some experience with this type of equipment.

A third method of creating a motive force through the membrane involves a two element wicking structure. The first of the two elements is the microporous membrane which serves as the reaction layer or retention layer for the test reagent-containing plastic beads. The second element is an absorbent member which is formed from a porous, liquid absorbent material. The liquid absorbent member is placed below and in contact with the lower surface of the microporous membrane, that is, the surface opposite the test surface or that surface which supports the plastic test reagent coated beads. A liquid sample placed in the reservoir contacts the upper surface of the membrane and begins to pass through and saturate the membrane. When the liquid reaches the lower surface of the membrane, it contacts the absorbent member and is rapidly drawn through the membrane due to a wicking or capillary action of the liquid absorbent member.

Although such two element diagnostic structures are effective in eliminating liquid from a test sample placed in a reservoir above the two element structure and are easy to use even by the layman, such devices suffer from certain drawbacks. Reproducible and uniform flow through the test or retaining microporous membrane is required if test results are to be consistent and reproducible. A rapid flow rate through the membrane is required if the typical test is to be of practical application. If irregular flow patterns develop in the membrane or if excessive flow times occur, false positive or negative results may be indicated. To achieve optimal flow characteristics, effective and uniform absorption across the lower gross surface is a requirement. However, there exists a critical problem with such two element structures which can impede effective absorption. This results from air or gas bubbles being trapped between the membrane layer and the pad of absorbent material, frequently in an irregular manner across the lower gross surface of the membrane. The formation of such air bubbles results from the inherent nature of the materials used and the structure of such a diagnostic device. In use, the liquid sample must first wet the membrane and thereafter flow through the depth of the membrane to contact the absorbent pad located therebelow and in contact with the membrane. The variations in intimacy of contact between the membrane and the absorbent pad and, perhaps, differences in the time at which the liquid front emerges from the membrane leads to liquid contacting the absorbent pad in some areas before others. As a result, air bubbles are often trapped between the membrane and pad during the initial wetting. Once trapped, these air bubbles cannot escape, and they effectively block liquid flow for the remainder of the test in the areas of the membrane superposed above them. Since such two element diagnostic devices rely solely on the vertical component of capillary action to draw the liquid from a sample through the membrane layer, any air trapped between the membrane and the absorbent pad can seriously impede, if not completely block, the flow of liquid through certain areas of the membrane layer. These blocked areas exhibit different flow characteristics than the rest of the membrane and may result in false positive, false negative, or indeterminant readings in a test.

In an attempt to overcome this problem and improve contact and communication between the membrane and the absorbent pad, modifications of such two element diagnostic structures have been tried. Thus, a three element or three layer device has been employed in which inter-layers of a fluffy fibrous material is placed intermediate the membrane and the absorbent pad, the purpose of the inter-layers being to fill and bridge any voids between the membrane and the absor bent pad. However, like the two element structures described above, intimacy between all layers is still requisite for proper function and may be compromised by slight differences in assembly from one device to the next. Insufficient compression of the layers may also result in poor communication between layers, even with the additional fluffy inter-layers interposed between the membrane and the absorbent pad. Furthermore, excessive compression can cause the membrane to bulge away from the lower structures, also resulting in similar degraded inter-layer communication.

A different problem also occurs with the membranes commonly employed with diagnostic test devices in current use. Many of the liquid samples tested with such diagnostic devices contain proteinaceous material. Many of the tests themselves depend upon reaction of a test reagent with a specific analyte which is typically a protein. The microporous membranes frequently used in such diagnostic devices have an affinity for proteinaceous materials and tend to absorb such materials present in the sample. The absorption is both specific and nonspecific. The absorption of nonspecific protein can lead to false positive results, thus making the test unreliable.

To avoid such absorption of nonspecific protein, a deactivating or blocking treatment is performed which renders the membrane inert or passive toward extraneous or nonspecific proteins in the liquid which is assayed. Although somewhat effective in minimizing nonspecific absorption of protein, the blocking treatment does have certain shortcomings. First, depending on the type of membrane being treated, technical difficulties may deter an even and thorough saturation of the membrane with blocking solution. In addition, substances used in the blocking treatment and in treating the membrane to minimize bacterial growth during storage are sometimes toxic and thereby present a health hazard to those people who are frequently exposed to the materials used in the blocking treatment, particularly those who are engaged in manufacturing operations.

A third problem which is occasionally encountered with the media used as microporous membranes in diagnostic devices results from the light emitting properties of some of the materials employed. Thus, some of these membrane materials fluoresce. Such fluorescence may interfere and give a spurious background when the membrane is viewed at the end of a test under light of a particular wavelength range used to detect a positive test.

3

The present invention provides a diagnostic device comprising: a receptacle having an open bottom; and a liquid absorbent, wettable, porous structure having a low non-specific affinity for amide group-containing substances, as measured by the Immersion Protein Binding Assay, secured to said receptacle and disposed across the open bottom. The liquid absorbent, wettable, porous structure may comprise a non-woven structure of thermoplastic, liquophilic fibers having a low affinity for amide group-containing substances. Further, the amide group-containing substances may comprise proteinaceous substances. The fibers may be hydrophilic and may be formed from a thermoplastic polyolefin, polyamide, polyester, or copolymer of at least one of the aforementioned combined with a surface active agent. The fibers may be formed from a surface active agent combined with a thermoplastic polymer of polyethylene or polypropylene or copolymers of ethylene or propylene and an $\alpha,\beta$-ethylenically unsaturated alkene having from 3 to 12 carbon atoms per molecule, e.g., the polyolefin may be a copolymer of polyethylene and 1-octene.

The fibers of the diagnostic device provided by the present invention may be microfibers having an average diameter in the range of 0.5 to 20 microns and said structure may be microporous. The surface active agent may be a mono-, di-, or triglyceride of a carboxylic acid. The carboxylic acid may be a fatty acid having 12 to 22 carbon atoms, and the surface active agent may be a monoester of Z-9-octadecenoic acid and 1,2,3-propanetriol.

The fibers of the diagnostic device provided by the present invention may further include an ultraviolet absorbing compound. The ultraviolet absorbing compound may comprise hydroxy substituted benzotriazole.

The present invention also provides for a porous medium for use in diagnostic devices comprising a liquid absorbent, non-woven material formed from thermoplastic, liquophilic fibers having a low non-specific affinity for amide group-containing substances as measured by the Immersion Protein Binding Assay.

The porous structures embodying the present invention have certain properties which are highly desirable in a diagnostic test device; namely, liquophilicity (and preferably hydrophilicity), i.e., being wettable by a liquid, high liquid absorbency, and low affinity for amide group-containing substances, particularly proteinaceous substances. In addition, the ability to control pore characteristics by selection of fiber diameters and web weights (thickness) as well as adjustment of manufacturing conditions, such as calendering, allows the production of uniform porous as well as graded or stepped porous structures which provide reproducible results and permit the selection of pore characteristics for particular applications, such as the retention of test reagent-containing beads.

A diagnostic device embodying the present invention may include a receptacle or reservoir, such as a tube or well, having a porous structure located at the bottom of the reservoir. The porous structure can serve either as a reaction zone or layer by incorporating a test reagent on its surface, or preferably, as a retention layer to support test reagent-containing particles, such as plastic spheres or beads, for example, polystyrene beads. The same porous structure also functions as a liquophilic, or wettable, liquid absorbent member. This porous structure, in the form of a nonwoven mat or web of fibers, provides a continuous pathway for consistent liquid flow communication between a first gross surface, such as a reaction surface or retention surface, and a second or opposite gross surface.

The fibrous structure is formed from a material which, although being liquid absorbent and readily wettable, has an inherent low affinity for binding amide group-containing materials, particularly proteinaceous type materials which may be measured by the Immersion Protein Binding Assay, described below. Thus, unlike conventional materials used for the same or similar purposes, the porous structure, or the nonwoven web (or mat) of medium forming same, requires no blocking to obviate protein absorption. Accordingly, a step which is generally required prior to use conventional diagnostic membranes or the devices in which they are incorporated is eliminated in embodiments of this invention.

In contrast to porous structures typically em ploying two elements, a microporous membrane and an absorbent member, the single element structure in embodiments of the present invention is formed by a generally simpler manufacturing process. Since a single element is employed, problems of mating two elements is eliminated in the assembly process which may then be more readily automated.

To attain the desirable low affinity for amide group-containing materials, particularly proteinaceous materials, and the liquophilic, preferably hydrophilic, nature of the porous absorbent structure, the fibers used to form the nonwoven structure are formed from a thermoplastic material which incorporates a surface active agent, such as a surfactant or wetting agent. Unlike many conventional nonwoven materials used for similar purposes in diagnostic devices which require binder to hold the fibers together, the porous structure in embodiments of the present invention, in which long fibers are mechanically entangled, is free of binder.

In addition, the porous structures in embodiments of the present invention and the fibers from which they are formed typically do not fluoresce or give a spurious background. However, to eliminate any residual fluorescence, an ultraviolet light absorbing compound may be combined with the polymer and wetting agent.

The wettability or liquophilicity of a solid structure, e.g., a membrane, is a function of that structure's critical surface energy and the surface tension of the applied liquid. If the critical surface energy is at least as high as the surface tension of the liquid, the liquid will spontaneously wet the solid structure, which may be termed "liquophilic" with respect to that liquid. For example, a porous membrane having a critical surface energy of 72 dynes/cm or higher will be wetted by water which has a surface tension of 72 dynes/cm and, therefore, tend to freely pass aqueous solutions, i.e., the membrane is "hydrophilic". The capability of a porous structure (membrane) to be wetted by a liquid can be determined by placing a drop of the liquid on the porous structure. The angle of contact provides a quantitative measure of wetting. A very high angle of contact indicates poor wetting and may be used to characterize a "liquophobic" material, while a zero angle of contact defines complete or perfect (liquophilic) wetting. Materials used in embodiments of the subject invention as the wettable or liquophilic porous absorbent structure are characterized by being readily or spontaneously wetted by the applied liquid and have a low angle of contact with the applied liquid. Indeed, when a drop of a test liquid(s) is placed on the wettable or liquophilic layer of the porous structure, the drop of liquid penetrates the layer and provides a low angle of contact with the upper surface of the porous structure.

As used herein, "analyte" refers to any substance or substances being analyzed for or determined by a diagnostic test. The presence of an analyte is typically indicated by the appropriate detection and/or measurement of a chemical or physical property after reaction or complex formation between the analyte and a detection reagent. Examples of properties which may be observed include color changes, radioactivity or enzyme reactions. The terms "reagent", "test reagent", "detection reagent", and like terms refer both to substances reacting directly with an analyte as well as to substances used to convert either another substance or the analyte to a substance suitable for providing an indication, typically an optical indication, of the presence of the analyte. The term includes simple chemical substances, including elements and compounds, of an ionic or molecular nature as well as more complex or macromolecular structures, such as proteinaceous materials, and includes substances of a biological nature such as antigens, antibodies, enzymes, antibody-enzyme conjugates, haptens, receptors, lectins, gene probes, and the like, as well as viruses and bacteria. The analyte may also be one or more of the substances falling within the definition of a reagent. In the case of antibodies, reagents and analytes may each be monoclonal or polyclonal antibodies. Additionally, the analyte may include drugs, peptides, cells, and organelles. A reagent may additionally include buffers, indicator molecules, and substrates.

The term "proteinaceous materials", as used herein, refers to compounds which contain a peptide bond or linkage and includes proteins and polypeptides. The term may also include substances not primarily proteins but which have sterically accessible amide moieties or polypeptide fragments.

Terms such as "surface", "porous structure surface", "media surface" or like terms, used in the singular or plural, are intended herein to include not only the gross surfaces, i.e., the external or outer surfaces, such as those which are exposed to view, but also the internal surfaces or those surfaces which define the pores of the porous structure or medium. Thus, the porous structure or medium surface is that portion of the porous structure or medium which is capable during use of being contacted by a fluid, particularly a liquid. As distinguished from the porous structure surface area, which refers to the area of both internal and external surfaces, the exposed planar dimensional area of the material (i.e., the area of the gross surface) is herein referred to as the porous structure area.

The terms "web" and "mat", as employed herein in describing the liquid absorbent, porous structures of the present invention, refer to non-woven porous structures in which the structural integrity is attributable to entanglement of fibers. Webs, which are frequently thinner than mats, are often formed from a single layer of fibers. Mats are typically formed from several layers of fibers or webs.

The terms "specific binding", "specific protein binding", and like terms, refer to bonds formed between members, typically proteinaceous members of a biospecific complex involving both molecular forces, other than covalent forces, and the complementary mating of configurationally commensurate portions of molecules. The terms "non-specific binding", "non-specific protein binding", "binding in a non-specific manner" and like terms, refer to bonds formed between a peptide group-containing material, preferably a proteinaceous material, and another substance involving the same type of molecular forces but lacking the configurational interactions found in specific binding.

A diagnostic device embodying the present invention can include what might be described as at least one receptacle or reservoir in which the walls of the receptacle are formed from a liquid impervious material, inert to solvents, reagents, and like materials used in the manufacture of the device or in diagnostic tests for which it is used. Typically an inert plastic, such as polystyrene, polyolefins (polyethylene and polypropylene, for example), or the like, is used. Defining the bottom of the receptacle is a liquid absorbent, porous structure. In its simplest form, the diagnostic device may simply be at least one tube having the porous structure disposed across and secured to one of the open ends of the tube. Other alternatives exist, however. For example, the porous absorbent structure may be disposed within the receptacle or tubular structure secured to the walls thereof such that upper and lower gross or external surfaces of the porous structure are at substantially right angles to the longitudinal axis of the tube. Such an arrangement permits the porous structure to be located below or recessed from the upstream or inlet opening of the reservoir but also, if desired, above and recessed from the outlet or downstream side of the diagnostic device, thereby permitting amounts of liquid exceeding the capacity of the porous, absorbent structure to be directed to a liquid receiver. Another alternative includes a housing having an exterior and including a chamber within the housing having one portion open to the exterior of the housing and another portion closed from the exterior of the housing and a porous, absorbent, wettable structure disposed across the open portion of the chamber, the porous structure having a first surface communicating with the exterior of the housing and a second surface communicating with the closed portion of the chamber. While the above described structures are preferred for the diagnostic devices embodying the present invention, this invention can be embodied by diagnostic devices having other structures, particularly those in which a well-type arrangement having a porous, liquid absorbent, liquophilic material defining the bottom wall of the well is employed.

Porous Structure:

The single element, porous, liquid absorbent, structure employed in a diagnostic device embodying the present invention is a nonwoven fibrous, preferably microfibrous, structure which is liquophilic (i.e., wettable). The structure is also, preferably, hydrophilic and has a low affinity for amide group-containing, particularly proteinaceous, materials.

The pore rating selected for the medium employed as the porous structure will depend on the thermoplastic material used to form the fibers or microfibers and the particular application to which the diagnostic test device will be put. Thus, if the porous absorbent structure will be used to support test reagent-containing plastic beads or the like, its expected primary application, the pore rating may be different than applications in which the diagnostic device will be employed with a test reagent immobilized in some manner within or, preferably, on the surface of the porous absorbent structure. The nature of the liquid sample being tested will in many instances also determine the pore rating of the porous structure to be used. Thus, if the sample contains significant solid particulate matter or is viscous, it may be desirable to use a mat with a larger pore rating. However, pore ratings are typically in the range of 0.5 to 200 microns, preferably the uncalendered medium has a pore rating of 5 to 20 microns, and the calendered porous structure has a pore rating of 1 to 5 microns. Pore ratings may also be varied across the thickness of the porous structure from one gross surface to the other. This may be achieved by means of a graded or stepped porous, liquid absorbent structure in which multiple webs are superposed on one another to form the composite porous structure. The pore rating of the porous structure may be controlled by calendering. When the same chemical composition is used throughout the stepped pore rating medium, other methods besides the preferred calendering may be used to obtain such media. Thus, variations in belt speed, rate of resin extrusion, resin supply pressure, and diameter of nozzle opening may be used. Although uncalendered porous structures are generally preferred because of higher liquid absorbency, a preferred embodiment of the present invention includes a porous "composite" unitary structure in which a calendered portion of the structure is located at or adjacent one gross surface of the porous medium and the remainder of the structure is uncalendered and inseparable therefrom. Typically, when viewed in cross-section, the calendered portion amounts to about 10-35% of the thickness of the web and is generally, preferably, less than 20%. The thickness of the mats in embodiments of the present invention may vary depending on the application. Typically, the thickness is not more than about 12mm (1/2 inch).

Absorbency of the porous structure is due primarily to the nature of the absorbing material employed and both the void volume and gross volume, or thickness, of the porous structure. The last two characteristics, void volume and gross volume, also known as "bulk characteristics", can be controlled in the manufacturing process to yield a uniform product which provides reproducible results. Individual webs may be produced having a "web weight" in the range of 5.4 to 107.6 grams/meter$^2$ (0.5 to 10 grams/foot$^2$) of medium and a void volume of 30 to 90%, preferably 50 to 80%. When preparing the porous, liquid absorbent, fibrous structures, several passes through the apparatus employed in their preparation creates a multi-layered or multi-web porous structure or mat which is, in most instances, the desirable form of the porous structure. The web weights or, alternatively, "mat weights" will, of course, depend on the thickness of the final porous structure which may be selected based on the application in which the mat is expected to be used. Suitable web weights for the mats of typically range from 21.5 to 4300 grams/meter$^2$ (2 to 400 grams/foot$^2$) of medium and web weights of 43 to 1076 grams/meter$^2$ (4 grams/foot$^2$ to 100 grams/foot$^2$) are preferred. Suitable void volumes for such layered mats are within the same ranges indicated above for the webs employed in the present invention. Manufacturing conditions employed to obtain such mats are discussed below.

As indicated above, the fibrous, and preferably microfibrous, media employed in embodiments of the present invention are liquid absorbent and quite wettable. The latter property is reflected by the Critical Wetting Surface Tension (CWST), which is the surface tension of a liquid that is just capable of wetting a porous structure. The microfibrous porous absorbent media can be prepared to provide suitable wettability for any liquid employed. However, since aqueous solutions are used most frequently in diagnostic tests, the porous structures exhibit CWST values suitably of at least 70 to 72 dynes/cm. Preferably, the porous structures have CWST values of at least 100 dynes/cm.

The absorbent fibrous structures also exhibit a low affinity for binding amide group-containing materials, particularly proteinaceous substances in a non-specific manner as measured by the Immersion Protein Binding Assay (IPBA), described below. When tested by this procedure, media which sorb no more than about 85 micrograms of amide group-containing material per cubic centimeter of media are considered, for purposes of the present invention, to have a low affinity for amide group-containing materials, such as proteinaceous materials. Preferred are very low affinity media, such as those media which sorb no more than about 50 micrograms of amide group-containing material per cubic centimeter of media. The media most preferred include those which bind below 30 micrograms of amide group-containing material per cubic centimeter of media. In this procedure, the affinity for amide group-containing materials is indicated by a test wherein protein solutions are applied to a sample of medium, nonbound excess protein flushed away, and the sample of medium assayed for bound residual protein.

The IPBA, described in greater detail below, is a radioimmunoassay. To conduct this test, uniform size (for example, 13 mm diameter round discs) samples are cut, identified, and placed in a suitable container along with a solution containing a known amount of unlabelled goat IgG and $^{125}$I-goat IgG in a buffered solution, such as phosphate buffered saline solution (PBS). The solution is counted with a gamma counter prior to contacting the membranes with the solution to determine the ratio of counts to unit of mass of the goat IgG. All of the samples are then placed in the PBS solution containing the labelled proteinaceous material and agitated for a fixed period of time, on the order of about one hour. The solution is then decanted from the samples and the remaining liquid removed by pipet. The media samples are then washed in a PBS solution of the same volume as the goat IgG solution, the samples being agitated in the solution for approximately 5 minutes. The washing procedure is repeated twice more, and the samples are removed from the wash solution, blotted, and counted with a gamma ray counter. The amount of protein bound by the membrane is then determined.

In addition to the weight per square foot and void volume, which can be controlled by calendering of the assembled fibrous mat, another important factor in determining pore characteristics is fiber diameter, discussed below.

Thermoplastic Fibers:

Many of the characteristics associated with the porous, liquid absorbent, wettable structures in embodiments of the present invention are attributable to the fibers employed to make these structures. These fibers are formed in major part from thermoplastic polymers and exhibit a low affinity for amide group-containing substances, particularly toward proteinaceous materials. Typically, the thermoplastic polymers used to form the fibers of the porous absorbent structure are liquophobic and, in particular, hydrophobic.

Materials suitable as the thermoplastic polymer are those materials which are generally inert toward the solvents, reagents, and other substances typically present either in analyte-containing samples or test reagent solutions used in particular diagnostic tests. Preferred are polyolefins, polyamides, polyesters, and copolymers of the aforementioned. Most preferred are polyolefins, particularly polyethylene and polypropylene and copolymers of ethylene or propylene, and other olefins. Such other olefins are preferably $\alpha,\beta$-ethyl enically unsaturated alkenes, more preferably those olefins having from 3 to 12 carbon atoms per molecule and most preferably ones with 4 to 8 carbon atoms per molecule. Exemplary of such other olefins is 1-octene. Preferred among the polyolefins and copolymers thereof are linear low density polymers, preferably linear low density polyethylene copolymers (LLDPE, described in U.S. Patent 4,578,414 to Sawyer et al. obtained by copolymerizing ethylene with other alpha-olefins.

As indicated above, the thermoplastic materials used in preparing the liquophilic fibers are in most instances liquophobic and typically hydrophobic. This is the case for those materials which are particularly preferred, the polyolefins. Such polymers, while having certain characteristics which make them useful in the present invention, because of their liquophobic or hydrophobic nature, are not well adapted, when in web or mat assemblies, to readily pass liquids, such as the type found in test samples. The fibers and porous structures formed therefrom must be wettable by the liquids which form the sample solutions or reagent solutions employed in diagnostic tests. Therefore, the CWST of the fibers must equal or exceed the critical surface tension of the liquid employed. In the present invention, this is achieved by combining a wetting or surface active agent with the thermoplastic polymer used to form the fibers.

The wettable fibers or filaments employed to form the absorbent, porous structures are prepared by compounding a thermoplastic polymer with at least one and preferably only one surface active agent. The term "surface active agent" is intended to include wetting agents and surfactants generally. However, the term is not meant to suggest that the surface active agent is formed only as a coating on the surface of the fibers. Rather, because of the way in which the thermoplastic polymer and surfactant are blended and the fibers are formed some of the surfactant is believed to be contained within the fibers. The preferred surface active agent is a mono-, di-, or tri-glyceride or a mixed glyceride and is preferably a mono-glyceride of a carboxylic acid, particularly a fatty acid. The acids employed may be saturated or unsaturated, preferably the latter. They have a length of about 12 to 22 carbon atoms, preferably about 14 to 18 carbon atoms. The acid most preferred is Z-9-octadecenoic acid and the surfactant most preferred is the mono-ester of Z-9-octadecenoic acid and 1,2,3-propanetriol (glycerol).

The surface active agent, or a mixture of such agents, is present in an amount of 0.1 to 5%, preferably 0.1 to 3% by weight, based on the total weight of the polymer formulation. When a polymer formulation which includes a polyolefin is used, particularly one which employs polyethylene or a copolymer of ethylene and the preferred $\alpha,\beta$-ethylenically unsaturated alkene, it is preferable to use 0.25 to 2%, by weight, of the surface active agent. Although higher amounts of the surface active agent may be used, it does not appear beneficial to do so and a greater expense is incurred. In addition, using higher amounts of the wetting agent runs the risk of incurring more than negligible extraction of wetting agent during a treatment or test procedure. Most preferably, about 1% of the wetting agent is employed when formulating the LLDPE polymer formulations used in preparing the fibers of the porous structure of embodiments of the present invention.

Although the porous absorbent structures and the fibers from which they are prepared show little tendency to fluoresce, any spurious background may be eliminated by combining an ultraviolet absorbing material with the polymer and surface active agent in the polymer formulation prior to formation of the fibers. Any ultraviolet absorbing material which is chemically inert with respect to the surface active agent and the polymeric material, as well as solvents and reagents, employed in diagnostic applications for which the porous structure may be used and which will not be leached from the fibers by typical solvents are suitable for use in the present application. Examples of suitable materials which may be used as the ultraviolet absorbing materials are listed in "Modern Plastics Encyclopedia", pp. 638 and 639, 1982-1983 edition. Preferred materials are those which are indicated as being recommended for the particular polymer employed in the polymeric formulations of embodiments of the present invention (as, for example, polyesters, polyethylene, and polypropylene). Most preferred are hydroxy-substituted benzotriazoles, many of which are available under the trademark TINUVIN™ (a registered trademark of Ciba-Geigy). Particularly preferred is 2-(3-t-butyl-2-hydroxy-5-methylphenyl)-5-chlorobenzotriazole, available from Ciba-Geigy as TINUVIN™ 326.

In formulating the compositions used to prepare the fibers, it is preferred to use concentrated master batches of blends of polymer and surface active agent which are subsequently blended, as portions, with additional quantities of barefoot (uncombined) polymer resin. This results in a polymer formulation which incorporates the surface active agent in a more dilute form directly in the bulk polymer resin. Alternatively, the surface active agent may be mixed in pure form with the polymer while the latter is in a molten state. The ultraviolet absorbing material may also be mixed while the thermoplastic polymer and wetting agent are being blended. Alternatively, the various additives may be added sequentially.

A preferred polymer formulation useful in preparing the fibers which form the nonwoven porous structure includes a copolymer formed, by weight, from a major portion (above about 90% and preferably greater than about 94%) ethylene and a minor amount (less than about 10% and preferably less than about 6%) of octene-1. Typically, this copolymer, containing no wetting agent, is mixed with a portion of copolymer containing about 5% of the preferred wetting agent, 1,2,3-propanetriol mono ester of Z-9-octadecenoic acid. The proportions of copolymer containing wetting agent and "pure" copolymer are mixed to provide the desired concentration of wetting agent.

8

The polymer formulation may be prepared by any convenient method typically used in compounding thermoplastic polymer formulations. The method suggested in U.S. Patent 4,578,414 may be used to advantage. Specifically, the surface active agent may be combined with the polymeric material when in a molten form by mixing the materials according to commonly used techniques and equipment. Examples of such techniques include roll-milling, mixing in a Banbury type mixer, or mixing in an extruder barrel, etc.

The porous absorbent webs are prepared in a melt-blowing procedure employing a melt-blowing apparatus. This process was first developed by the Naval Research Laboratory and is described in "Manufacture of Superfine Organic Fibers", U.S. Department of Commerce, Publication PB111437 (1954) and in Van A. Wente, "Superfine Thermoplastic Fibers" (August 1956). In such process, the polymeric material or, more preferably, the polymeric resin which includes the blended wetting agent is heated until molten and extruded through multiple nozzles under pressure. A stream of hot air is directed toward the emerging molten material to entrain the emerging fine fibers. Control of fiber size is achieved as a result of manipulating the resin pressure, the air pressure or velocity, and the temperature. The conditions are varied to obtain microfiber diameters preferable in the present invention. Typically the fiber diameters are on the order of 0.5 to 20 microns, preferably 1 to 8 microns.

The apparatus is adjusted so that the microfibers are directed to impact on a collection surface to form a web. As the fibers strike the collection surface they become mechanically intertwined with one another, forming a coherent mass of microfibers of great uniformity. The conditions are adjusted so that little if any fusion occurs between the individual microfibers, since excessive fusion results in webs which are undesirably stiff and brittle and create problems in manufacture and later handling of the web.

Typically, the collection surface is a moving belt formed from a material which exhibits little adhesion to the microfibers that impact on its surface. Alternatively, a substrate for the microfibers may be employed which permits the microfibers to intertwine with the substrate, producing a web which has a greater strength than the microfibers alone. Such substrates may be composed of substances which, like the thermoplastic resins combined with the wetting agents, are chemically inert to the solvents, test reagents, or components found in either the analyte or test solutions or solvents employed in the manufacturing or testing processes. Examples of suitable substrates include polyesters and polyolefins, and may be in the form of a woven or nonwoven matrix. The substrate may also be another web or mat of the porous absorbent medium formed of the same or different material. Typically the material is chemically the same but the pore rating is different. The latter characteristic is generally achieved by calendering. Thus, a stepped or graded pore rating may be provided in a composite medium.

The belt speed, in combination with the rate of extrusion of the resin and the velocity of the airstream, determines the web weight per square foot. Web weights in the range of 1.08 to 108 grams/meter$^2$ (0.1 to 10 grams/foot$^2$) of medium are typically obtained in a first pass through the apparatus when the first layer is obtained. Multiple passes are preferred to both increase the web weight and enhance uniformity of the resultant mat. Most preferred are mats in which the fibers impact on the previously formed web or layer with the opposite orientation (left for right or 180° from the first orientation) during subsequent passes. With repeated passes, virtually any desired web weight can be achieved for the finished product. In the present invention, web weights of the mats of 21.5 to 4306 grams/meter$^2$ (2 to 400 grams/foot$^2$) of medium are preferred and most preferred are web weights of 43.1 to 1076 grams/meter$^2$ (4 to 100 grams/foot$^2$).

In some cases, it may be desirable to provide a graded surface wherein the upper surface of the web or mat is coarser and the lower layers are finer. This structure may be used in some situations where enhanced flow is desirable when the liquids being applied contain particulate contamination which tends to block the surface. Alternatively, and typically more preferable, the fine face of the porous structure can be arranged uppermost in the diagnostic device when it is desirable to retain all particles on the surface but simultaneously reduce the resistance to flow in the lower structure. Such embodiments can be achieved by variation in the resin supply pressure, the nozzles employed and/or the air pressure during web formation. This results in variations in the diameter of the fibers.

As indicated above, the pore rating and void volume may be altered by compression of the porous, absorbent structure. This is done most conveniently by a calendering process employing heat and pressure in which the porous structure is passed between heated rolls. Preferred is an apparatus in which one roll is steel and the other is compressed cotton. A temperature is used which is just below the softening point of the thermoplastic polymeric material. The objective is to employ a combination of temperature and pressure which will result in a permanent deformation of the fibers but will not produce significant fusion and fiber-to-fiber bonding, thereby resulting in a mat in which the integrity is determined by mechanically intertwined fiber rather than bonded or fused fibers.

Moreover, this procedure may be employed to form a preferred embodiment of the present invention, a unitary, composite, partially calendered, porous absorbent structure, described above. In forming the composite mat, a previously calendered web may be reintroduced to the melt-blowing apparatus to receive additional fibers, and layers of microfibers deposited thereon. The resulting structure has, in section, a calendered "layer" or section which is unitary with and inseparable from the remaining uncalendered layer or section of the composite, porous, absorbent structure. The calendered layer is arranged in a preferred embodiment of the diagnostic device of the present invention as a top face which although less absorbent possesses a restricted pore size that, for instance, can enhance the capture or retention of antibody coated latex beads. The bulk of the remaining microfibers which form the lower section of the porous, absorbent structure are optimized for liquid flow and liquid absorption.

The present invention may be employed to produce uniform, porous, absorbent webs which may be used in diagnostic devices to produce uniform and reproducible test results. Such uniformity in the mats produced and the concomitant test results is determined in part by the ability to control fiber diameters and the production of uniform fiber diameters in any or all of the medium. The uniformity of mats also results from the production of consistent web weights, that is, mats having reproducible and uniform densities as determined by taking a cross-sectional sample from any part of a roll of media which may be compared to a sample taken from another part of the roll. This is achieved by multiple laydowns or passes through the apparatus in which the fibers are extruded.

When test reagent-containing beads are employed in the test device, test results (sensitivity) may be improved by concentrating the signal indicating material (test reagent) at or near the surface of the mat. This is achieved by compression of the mat with calibrating pressure at elevated temperatures, to appropriately set the pore rating. The extent of calendering is determined with the size of the parti cles in mind. Thus by appropriately selecting calendering one may improve the sensitivity or bead-holding ability; however, void volume and absorbency, i.e., the ability of the structure to hold liquid may be lost. As indicated above, both effects, particle retention and liquid retention, may be achieved to any desired degree with little sacrifice of the other by calendering a first layer and then laying down other uncalendered layers on the first layer. This permits the calendered layer to retain particles and the non-calendered layers to provide liquid absorbency.

Example 1: Preparation of a Wettable Polypropylene Mat

A wettable polypropylene was prepared containing 99.5%, by weight, of Exxon polypropylene (grade 3045) and 0.5%, by weight, of Atmer 645 wetting agent. The latter is a complex mixed glyceride with a long chain (12 to 16 carbon atoms) fatty acid adduct, available from ICI America Inc. The mixture was prepared by blending 18.14 kgs. (40 pounds) of Exxon polypropylene, with 0.11 kgs. (0.25 pounds) of Atmer 645 wetting agent. This mixture was tumbled in a rotating drug mixer for 30 minutes to produce pellets, which contained the wetting agent on the surface thereof.

These pellets were heated to 343.3° C (650° F), and fiberized by passage through a melt blowing die. The resin pressure was 140,620 kg/square meter (200 psi) and the air pressure 15,468 kgs/square meter (22 psi). The emerging fibers were collected on a polypropylene non-woven substrate (Lutrasil 5020). Fibrous webs with weights of 21.5, 43.1 and 86.1 grams/meter$^2$ (2, 4 and 8 grams/foot$^2$) were collected. The non-woven substrate was stripped from the web prior to tests and use.

The CWST of these webs were measured and found to be 72 dynes/cm. The wetting point for water requires a CWST of 72 dynes/cm, which means that the webs were just water wettable. A web prepared from Exxon polypropylene alone measured 28 to 31 dynes/cm, and thus would not wet with water.

Example 2: Preparation of a Wettable Mat of Polyethylene and Octene-1

A wettable polyethylene was prepared from a copolymer of, by weight, 94% ethylene and 6% octene-1 (the copolymer, available commercially from Dow Chemical Company as Aspun 6809). To this copolymer was added 1%, by weight, of a monoester of Z-9-octadecenoic acid and 1,2,3-propanetriol as a wetting agent (commercially available as XU 61518.10 from Dow Chemical Company).

This mixture was prepared by a commercial compounder who blended 80 parts of Aspun 6809 with 20 parts of a stock concentrate, the total weight being 226.8 kgs. (500 pounds). (The stock concentrate is itself a mixture of 95% Aspun 6809 and 5% of the monoester of Z-9-octadecenoic acid with 1,2,3-propanetriol.) Thus the final composition of the mixture was 99% Aspun 6809 with 1% wetting agent.

Mixing was accomplished by first tumbling the components in a Banbury Mixer for 30 minutes. The mixture was then turned into a homogeneous blend by passage through an extruder at a temperature just above the melting point of the thermoplastic polymer, at about 205°C (400°F). (This low temperature is preferred to minimize degradation.) The extradite was then cooled and chopped into pellets, rebagged and returned.

The returned pellets were then heated to 321°C (610°F) and fiberized by passing through a melt blowing die at 150,465 kg/square meter (150 psi) resin pressure and 14,062 kgs/square meter (20 psi) air pressure. The emerging fibers were collected on a non-woven polypropylene substrate (Lutrasil 5020). In this manner webs of 21.5, 43.1, and 86.1 grams/meter$^2$ (2, 4, and 8 grams/foot$^2$) were prepared. Prior to test and use, the substrate was stripped from the webs.

Measurements were made on webs prepared from the copolymer of ethylene and octene-1 alone (method as above omitting the addition of the wetting agent), and yielded a CWST of 35 dynes/cm. The copolymer with wetting agent, described above, resulted in mats with a CWST of 104 to 115 dynes/cm. Water absorption in these mats was virtually instantaneous.

## Example 3: Calendering of Hydrophilic Medium

The melt blown webs were calendered by passing the web through a pair of heated rolls. One of the rolls is highly polished steel, and is heated by a hot oil circulation system. The second roll, in intimate contact with the first, is provided with a compressed cotton outer surface. Temperature and pressure regulating systems allow a wide range of heat and pressure to be applied to the pair of rolls.

The polyethylene web described in Example 2 was calendered at a roll temperature of 93°C and a pressure of 20 tons. The web speed was 1.49 meters/minute (16 feet/minute) through the nip, or intersection, of the calendering rolls. When tested for water bubble point (a measure related to the pore size of the web), the 86.1 grams/meter$^2$ (8 gram/square foot) sample went from a pre-calendered 330.2-381 mm (13-15 inches) water bubble point to approximately 1016 mm (40 inches) water bubble point. This indicated that the calendering had considerably decreased the pore size.

## Example 4: Measurement of Protein Binding:

The microporous, hydrophilic structures prepared according to Examples 2 and 3 as well as known nylon membranes were tested for their abilities to bind the protein goat immunoglobulin G (goat IgG) according to the general procedure for measuring protein binding outlined below.

### Immersion Protein Binding Assay

To determine the affinity of microporous wettable webs for protein containing materials, a radioimmunoassay was performed on several samples of webs prepared in Examples 2 and 3. In addition, the same procedure was used to determine the affinity of nylon membranes for protein containing materials. Included in the nylon membrane group were nylon membranes chemically activated (modified to enhance protein attachment) and those having surfaces modified to minimize affinity for protein.

To perform the test, 13 mm diameter disks were punched from each sample using a cork borer. For identification, nylon disks were labelled by marking with a pencil and disks of hydrophilic polyethylene webs were notched with a scissor. Two disks per sample were used.

A solution was prepared by combining 10 µg/ml of goat IgG (Sigma Chemical Company) and a solution of $^{125}$I-goat IgG (New England Nuclear Corp.) in phosphate buffered saline solution (PBS). This mixture was counted in a gamma counter and yielded a count of 50681 counts/minute/ml. Dividing by the mixture concentration of 10 µg/ml gives 5068 counts/minute/µg.

The biological protein material in PBS was adjusted to a pH of 7.0 with a solution containing 0.15 moles/liter sodium chloride and 0.02 moles/liter of a mixture of mono-, di-, and trisodium phosphate. In those situations in which more than one sample of media was tested at the same time, all samples of media were immersed in the same portion of solution and the volume of the total solution adjusted to provide 2 ml of solution per 13 mm disc.

The discs were agitated in the solution for a period of one hour, after which the IgG solution was decanted and the remaining liquid was removed from the immersion vessel using a pipet. PBS solution was added to the immersion vessel in the same volume as the previously used biological material-containing solution (2 ml per disc) and the membranes were agitated in the solution for 5 minutes to wash and remove residual protein. The wash solution was decanted and the washing procedure was repeated twice using fresh PBS solution each time for a total of three washes. The membrane discs were then removed, blotted gently between absorbent paper, and the residual activity was counted using an LKB Wallac Mini Gamma Ray Counter, model no. 1275. The amount of radioactive material on the disc reflects the total protein bound to the discs, i.e., both covalently bound protein in those discs which were chemically active and that which is present because of strong non-specific absorption.

Protein bound/unit volume of membrane was calculated by dividing the counts obtained after the PBS wash by 5068 counts/minute/$\mu$g, then dividing by the volume of the individual 13 mm diameter disks. Volume is calculated by the formula: $(\pi \times d^2)/4 \times$ thickness of the sample.

The data collected and calculated as described above are as follows:

Table 1

| Sample | Mat Weight (g/m$^2$) | Thickness (cm) | Counts | Protein Bound/unit volume of Membrane ($\mu$g/cm$^3$) |
|---|---|---|---|---|
| A | 43.1 | 0.015 [a] | 2510 | 25 |
| B | 86.1 | 0.025 [a] | 3909 | 23 |
| C | 43.1 | 0.033 | 4400 | 20 |
| D | 86.1 | 0.064 | 9326 | 22 |
| ImmunodyneTM [b] | | 0.0165 | 29607 | 267 |
| BiodyneTM A [c] | | 0.0165 | 34585 | 312 |
| LoProdyneTM [d] | | 0.0165 | 9443 | 85 |
| Blocked[e] ImmunodyneTM | | 0.0165 | 2822 | 25 |
| Blocked[e] BiodyneTM A | | 0.0165 | 2903 | 26 |
| Blocked[e] LoProdyneTM | | 0.0165 | 2545 | 23 |

[a] calendered

[b] ImmunodyneTM membranes are nylon membranes (available from Pall Corporation) surface modified to be chemically active for formation of covalent bonds with proteinaceous materials.

[c] BiodyneTM A (available from Pall Corporation) membranes are nylon 66 membranes which bind protein non-specifically.

[d] LoProdyneTM (available from Pall Corporation) membranes are surface modified membranes modified to minimize the binding of proteins.

[e] The membranes which are indicated as "blocked" have been chemically treated with a 0.5% casein solution prior to contact with the goat IgG solution to inactivate the structures to protein absorption

Example 5: Determination of Absorbent Capacity (Absorbency) of Polyethylene Webs

In this example, two rectangular pieces were cut from each of five samples: A, B, C, D (all polyethylene melt blown webs), and a sample of BiodyneTM A nylon membrane. For all samples, the length and width were measured, along with the thickness. For greatest accuracy, the melt blown webs were measured by noting the minimum setting on a micrometer that allowed the web to be drawn through the jaws of the micrometer without dragging. For all samples dry weights were determined.

To determine the volume of water that could be held per cubic centimeter of web volume, the following calculation was performed: The volume the fibers alone occupy is subtracted from the volume of the web (membrane) sample (length x width x thickness). The volume of the fibers alone is the dry web weight divided by the density of the polyethylene fibers (0.93 gms/cm$^3$ for Aspun 6809). The difference between the volume of the web sample and the volume of the fibers alone equals the void volume of the sample material. Since these fibers are highly wettable, absorbing water immediately upon contact therewith, the void volume of the medium is essentially equal to the retained water volume. The retained water volume divided by the volume of the web sample equals the amount of water that can be retained per cubic centimeter of medium. The calculation for the above sample A of wettable polyethylene indicates a sample

EP 0 394 021 A2

void volume which equals the retained water volume of 0.728 cm$^3$.

The Absorbency is the ratio of the retained water volume divided by the volume of the membrane. For a 1.082 cm$^3$ sample of the above-described polyethylene sample, an Absorbency was determined to be 0.673 cm$^3$ water/cm$^3$ web. For comparison, a similar determination was done for a 0.45 micron pore rated supported Biodyne™ A nylon 66 membrane. For a 1.236 cm$^3$ sample having a density of 1.10 gms/cm$^3$ and a polyester support having a density of 1.39 gms/cm$^3$, an average density of 1.24 gm/cm$^3$ was used. The sample void volume was 0.792 cm$^3$ and the Absorbency of the hydrophilic material was determined to be 0.64 cm$^3$ water/cm$^3$ membrane. The data derived from these determinations are listed below:

Table 2

| Sample | Mat Weight (g/m$^2$) | Retained Water Volume/Volume of Medium (cm$^3$ water/cm$^3$ medium) |
|---|---|---|
| A Calendered | 43.1 | 0.67 |
| B Calendered | 86.1 | 0.63 |
| C Uncalendered | 43.1 | 0.85 |
| D Uncalendered | 86.1 | 0.85 |
| Biodyne™ A | | 0.64 |

The term "uncalendered" as used in these examples refers to the material as laid down in the melt-blowing apparatus. Material indicated as "calendered" includes samples obtained by compressing webs taken from the same rolls as Samples A and B. Calendering was performed as described in Example 3. The Biodyne™ A nylon membrane is included for reference purposes.

As may be noted in Table 2, calendering of the material of Samples C and D to produce the material of Samples A and B results in a decrease in Absorbency while decreasing the pore rating. An Absorbency suitable for many applications of the present invention includes values greater than about 0.6 cm$^3$ water/cm$^3$ medium. Preferred, however, are values of at least about 0.8 cm$^3$ water/cm$^3$ medium. In many applications of such material in diagnostic test devices, particularly those which are used in conjunction with test reagent-containing beads, a composite, porous (preferably microporous), liquophilic (preferably hydrophilic), liquid absorbent (preferably water-absorbent) medium having a low affinity for amide group-containing materials (preferably a low binding of proteinaceous materials), as described above, may be used. Although the present invention includes as preferred embodiments graded, porous, liquid absorbent structures having multiple steps (up to as many as about 30 steps), an example of a most preferred material includes a medium having at one planar surface (intended as the upper surface in the device) a portion or layer, typically not more than about 35% of the cross-sectional area of the composite medium, of a calendered mat at the other surface (intended as the lower or downstream surface in the device) and constituting the remainder of the porous structure is an uncalendered (compressed) or lightly calendered portion of the same medium. The two portions of the composite membrane may be of the same or different chemical composition web (mat) weight. For ease of manufacture, the two portions of the medium are preferably of the same chemical composition and web weight.

## Claims

1. A diagnostic device comprising:
a receptacle having an open bottom; and
a wettable, liquid absorbent, porous structure having a low non-specific affinity for amide group-containing substances, as measured by the Immersion Protein Binding Assay, secured to said receptacle and disposed across the open bottom.

2. A diagnostic device according to claim 1 wherein said wettable, liquid absorbent, porous structure comprises a non-woven structure of thermoplastic, liquophilic fibers having a low affinity for amide group-containing substances.

3. A porous medium for use in diagnostic devices comprising a non-woven, liquid absorbent, porous structure formed from thermoplastic, liquophilic fibers having a low non-specific affinity for amide group-containing substances as measured by the Immersion Protein Binding Assay.

4. A diagnostic device according to claim 2 or a porous medium according to claim 3 wherein said fibers are formed from a surface active agent combined with a thermoplastic polyolefin, polyamide, polyester, or a copolymer derived from monomers used to form the aforementioned.

5. A diagnostic device according to claim 2 or a porous medium according to claim 3 wherein said fibers are formed from a surface active agent combined with a thermoplastic polymer of polyethylene or polypropylene or copolymers of ethylene or propylene and an $\alpha,\beta$-ethylenically unsaturated alkene having from 3 to 12 carbon atoms per molecule.

6. A diagnostic device according to claim 5 or a porous medium according to claim 5 wherein said polyolefin is a copolymer of ethylene and 1-octene.

7. A diagnostic device according to claim 2 or a porous medium according to claim 3 wherein said fibers are microfibers having an average diameter in the range of 0.5 to 20 microns and said structure is microporous.

8. A diagnostic device according to claim 4 or a porous medium according to claim 4 wherein said surface active agent is a mono-, di-, or triglyceride of a carboxylic acid.

9. A diagnostic device according to claim 8 or a porous medium according to claim 8 wherein said carboxylic acid is a fatty acid having 12 to 22 carbon atoms.

10. A diagnostic device according to claim 2 or a porous medium according to claim 3 wherein said fibers further include an ultraviolet absorbing compound.

11. A diagnostic device according to claim 2 or a porous medium according to claim 3 wherein said porous structure has a non-specific binding of amide group- containing substances measured by the Immersion Protein Binding Assay of no more than about 85 $\mu$g of amide group-containing substance/cm$^3$ of porous structure.

12. A diagnostic device according to claim 2 or a porous medium according to claim 3 wherein said porous structure has a non-specific binding of amide group-containing substances measured by the Immersion Protein Binding Assay of no more than about 30 $\mu$g of amide group-containing substance/cm$^3$ of porous structure.

13. A diagnostic device according to claim 2 or a porous medium according to claim 3 wherein the CWST of said liquid absorbent, porous structure is at least 100 dynes/cm.

14. A diagnostic device according to claim 2 or a porous medium according to claim 3 wherein said liquid absorbent, porous structure has an Absorbency of at least about 0.60 cm$^3$ of water/cm$^3$ of media.

15. A diagnostic device according to claim 2 or a porous medium according to claim 3 wherein said liquid absorbent, porous structure has an Absorbency of at least about 0.80 cm$^3$ of water/cm$^3$ of media.

16. A diagnostic device according to claim 2 or a porous medium according to claim 3 wherein said porous structure has pore dimensions varying stepwise between a first surface and a second parallel surface.

17. A diagnostic device according to claim 16 or a porous medium according to claim 16 wherein the porous structure includes a calendered portion at said first surface and an uncalendered portion at said second surface.

18. A diagnostic device according to claim 17 wherein said first surface comprises an upper surface and said second surface comprises a lower surface.

19. A diagnostic device according to claim 1 wherein said diagnostic device is adapted to be used in conjunction with a test reagent.

20. A diagnostic device according to claim 1 wherein said wettable, liquid absorbent, porous structure comprises a non-woven microporous structure of thermoplastic, hydrophilic fibers formed from a copolymer of polyethylene and 1-octene combined with a monoester of Z-9-octadecenoic acid and 1,2,3-propanetriol and having an average diameter in the range of 0.5 to 20 microns, a non-specific binding of amide group-containing substances of no more than about 30 $\mu$g of amide group-containing substance/cm$^3$ of porous structure, a CWST of at least 100 dynes/cm, and an Absorbency of at least a major portion of said porous structure of at least 0.80 cm$^3$ of medium.

21. A porous medium according to claim 3 wherein said liquid absorbent, porous structure comprises a non-woven microporous structure of thermoplastic, hydrophilic fibers formed from a copolymer of polyethylene and 1-octene combined with a monoester of Z-9-octadecenoic acid and 1,2,3-propanetriol and having an average diameter in the range of 0.5 to 20 microns, a non-specific binding of amide group-containing substances of no more than about 30 $\mu$g of amide group-containing substance/cm$^3$ of porous structure, a CWST of at least 100 dynes/cm, and an Absorbency of at least a major portion of said porous structure of at least 0.80 cm$^3$ of medium.

14